# EUROPEAN PATENT APPLICATION

(11) **EP 4 847 003 A1**
(43) Date of publication of application: **02.12.2026**
(21) Application number: 26179483.8
(22) Date of filing: 19.05.2026
(51) Int. Cl.: A61K 8/14, A61K 8/34, A61K 8/55, A61K 8/63, A61Q 19/00

(54) **HIGH-STABILITY NANOLIPOSOME AND MANUFACTURING METHOD THEREOF**

(30) Priority: 30.05.2025 KR 20250071437; 17.03.2026 KR 20260047557
(71) Applicant: Amorepacific Corporation, Seoul 04386 (KR)
(72) Inventor: SONG, Chaeyeon, 17074 Yongin-si, Gyeonggi-do (KR); HAN, Youngkyu, 17074 Yongin-si, Gyeonggi-do (KR); KIM, Suil, 17074 Yongin-si, Gyeonggi-do (KR); PI, Bongsoo, 17074 Yongin-si, Gyeonggi-do (KR); LEE, Somi, 17074 Yongin-si, Gyeonggi-do (KR); LEE, Eun Soo, 17074 Yongin-si, Gyeonggi-do (KR); CHOI, Joonho, 17074 Yongin-si, Gyeonggi-do (KR); HWANG, Jeongah, 17074 Yongin-si, Gyeonggi-do (KR); PARK, Wonseok, 17074 Yongin-si, Gyeonggi-do (KR); BAEK, Heungsoo, 17074 Yongin-si, Gyeonggi-do (KR); YOU, Jaewon, 17074 Yongin-si, Gyeonggi-do (KR)
(74) Representative: Viering, Jentschura & Partner mbB Patent- und Rechtsanwälte

(57) **Abstract**

The present disclosure relates to a high-stability nanoliposome and a manufacturing method thereof. Specifically, the present disclosure relates to a nanoliposome, including, as membrane components of the nanoliposome, a phospholipid; cholesterol; a polyol.

## Description

### BACKGROUND OF THE INVENTION

### Field of the Invention

The present disclosure relates to a high-stability nanoliposome and a manufacturing method thereof.

### Description of the Related Art

In modern pharmaceutical and cosmetic industries, accurate and effective delivery of an active substance (efficacy ingredient) is acting as an important factor for product performance and consumer satisfaction. In a pharmaceutical field, various drug delivery systems are being developed for a drug to reach a target site within a living body to maximize a therapeutic effect and minimize a side effect, and in a cosmetic field, various technologies are being researched so that an effective ingredient effectively passes through a skin barrier and reaches deep into skin, thereby exerting a beauty or therapeutic effect.

Among such delivery systems, a liposome is a lipid-based self-assembled structure and has excellent biocompatibility and biodegradability, and especially, due to an advantage that both water-soluble and fat-soluble substances can be loaded, the liposome is being actively utilized in the pharmaceutical and cosmetic industries. In a pharmacy field, drug delivery using a liposome is being applied to treatment of various diseases, and in a cosmetic field, a liposome is widely used in terms of skin penetration enhancement and stabilization of an efficacy ingredient.

However, when a particle size of a liposome is generally 150 to 200 nm or more, there is a limitation that skin permeability is lowered because it does not effectively pass through a stratum corneum of skin. The stratum corneum of skin is composed of a dense lipid-protein structure having a thickness of about 10 to 20 µm, and thus acts as a strong barrier that inhibits penetration of an external substance. Accordingly, as a measure for improving skin permeability of a liposome, various attempts for reducing a particle size of a liposome have been made.

However, due to a curvature limit of a phospholipid bilayer structure constituting a liposome, a physical constraint follows in implementing a particle size below a certain level, and a limitation also exists in stably manufacturing and maintaining a nano-sized liposome. Also, since a liposome is a self-assembled structure that sensitively reacts to external conditions such as concentration, temperature, and pH, a structure becomes unstable according to a change in environmental conditions, and due to this, a problem in which a loaded efficacy ingredient may leak may occur.

Along with this, a conventional liposome manufacturing method has problems that a process is complicated and a production unit price is high. For example, thin-film hydration, ultrasonication, and the like have a large energy consumption in a manufacturing process, and a quality deviation between batches easily occurs, so there is a limitation in mass production. Therefore, for practical commercialization of a liposome-based delivery system, precise control of a particle size, securing of uniformity of size, improvement of structural stability, and simplification of a manufacturing process and cost reduction are being recognized as technical tasks to be performed in parallel.

### SUMMARY OF THE INVENTION

The present disclosure is directed to providing a high-stability nanoliposome and a manufacturing method thereof, which can improve resistance against thermal and chemical stress by effectively controlling a membrane structure affecting a particle size, stability, and a loading rate of an efficacy ingredient of a liposome, and enable high-efficiency encapsulation and release control, and at the same time, can promote simplification of a process and easiness of mass production.

To achieve the above object, one embodiment of the present invention provides a nanoliposome, including, as membrane components of the nanoliposome, a phospholipid; cholesterol; a polyol; and a triterpenoid compound.

Also, one embodiment of the present invention provides a manufacturing method of the nanoliposome, including: a first mixing step of mixing a phospholipid, cholesterol, a polyol, and a triterpenoid compound; and a second mixing step of mixing the mixture with water.

The high-stability nanoliposome and the manufacturing method thereof according to the present disclosure can improve resistance against thermal and chemical stress by effectively controlling a membrane structure affecting a particle size, stability, and a loading rate of an efficacy ingredient of a liposome, and enable high-efficiency encapsulation and release control, and at the same time, can promote simplification of a process and easiness of mass production.

### BRIEF DESCRIPTION OF THE DRAWINGS

FIGS. 1A and 1B are views showing photographs of particle sizes of a conventional general nanoliposome (FIG. 1A) and a nanoliposome according to one embodiment of the present invention (FIG. 1B).
FIGS. 2A to 2D show results of confirming a skin stratum corneum permeation effect according to Test Example 1.
FIGS. 3A to 3D show results of a skin permeation test for a skin biopsy tissue according to Test Example 2.
FIGS. 4A and 4B show results of a membrane structure stability test according to Test Example 3.
FIGS. 5A to 5F show results of a membrane structure stability test under a severe condition according to Test Example 4.
FIGS. 6A to 6C show results of a cell efficacy confirmation test according to release of an efficacy ingredient according to Test Example 5.
FIGS. 7A and 7B show results of a structural transition confirmation test of a nanoliposome according to an amount of water added according to Test Example 7.
FIG. 8 shows a result (SAXS) of a nanostructure change confirmation test of a nanoliposome according to water content according to Test Example 8.
FIG. 9 shows a result of a morphology change confirmation test of a triterpenoid-containing GUV under an osmotic pressure condition according to Test Example 9.
FIGS. 10A to 10C show results of a molecular arrangement confirmation test of a triterpenoid within a membrane through GIWAXS analysis according to Test Example 10.
FIGS. 11A to 11D show results of a behavior confirmation test of a triterpenoid within a membrane through molecular dynamics (MD) simulation according to Test Example 11.
FIGS. 12A and 12B show results of a lateral diffusion restriction confirmation test of a triterpenoid-containing membrane through FRAP analysis according to Test Example 12.
FIG. 13 shows a result of an efficacy substance loading thermal stability confirmation test through a calcein leakage test according to Test Example 13.
FIGS. 14A and 14B show results of a long-term stability and freeze-thaw stability confirmation test according to Test Example 14.
FIG. 15 shows a result of an encapsulation efficiency and loading rate confirmation test of an efficacy substance according to Test Example 15.
FIGS. 16A and 16B show results of a cytotoxicity confirmation test according to Test Example 16.
FIG. 17 shows a result of a membrane thickness change confirmation test according to oil addition according to Test Example 17.
FIG. 18 shows a result of a SAXS-based nanoliposome structural stability confirmation test according to surfactant treatment according to Test Example 18.
FIG. 19 shows a schematic diagram of a polyol-based osmotic pressure-driven fragmentation mechanism.
FIG. 20 shows a curvature-based theoretical framework explaining size selection of a tubular structure (tubule) and a budding structure (budding).
FIG. 21 shows a result of a stability confirmation test of a Compound K-encapsulated nanoliposome according to Test Example 19.

### DETAILED DESCRIPTION OF THE INVENTION

Hereinafter, the present invention will be described in detail.

The present invention, in one aspect, may relate to a nanoliposome.

In one embodiment, the nanoliposome, as membrane components, may include a phospholipid; cholesterol; a polyol; and a triterpenoid compound.

In one embodiment, the nanoliposome, as a membrane component, may further include an oil.

In one embodiment, the nanoliposome, as membrane components, may consist of a phospholipid; cholesterol; a polyol; and a triterpenoid compound.

In one embodiment, the nanoliposome, as membrane components, may consist of a phospholipid; cholesterol; a polyol; an oil; and a triterpenoid compound.

In one embodiment, a particle size of the nanoliposome may be less than 30 nm.

In one embodiment, at least 80%, 85%, 90%, 95%, or 99% or more of particles of the nanoliposome may be less than 30 nm.

Specifically, at least 80%, 85%, 90%, 95%, or 99% or more of particles of the nanoliposome may be less than 30 nm, 29 nm or less, 28 nm or less, 27 nm or less, 26 nm or less, 25 nm or less, 24 nm or less, 23 nm or less, 22 nm or less, 21 nm or less, 20 nm or less, 19 nm or less, or 18 nm or less, and also, at least 80%, 85%, 90%, 95%, or 99% or more of particles of the nanoliposome may be 10 nm or more, 11 nm or more, 12 nm or more, 13 nm or more, 14 nm or more, 15 nm or more, 16 nm or more, or 17 nm or more. Such a uniform ultrafine nano-level particle size makes it possible to effectively pass through a dense lipid structure of a stratum corneum of skin, and thus may remarkably improve skin permeability of the nanoliposome.

**In** one embodiment, the triterpenoid compound may be one or more selected from among a natural triterpenoid, a semi-synthetic triterpenoid, and a synthetic triterpenoid. Specifically, it may be used in a form of a plant extract (e.g., Centella asiatica extract, licorice extract, etc.) itself including a triterpenoid compound or a mixture of triterpenoid compounds separated from the plant extract.

The triterpenoid compound may be one or more selected from among asiaticoside, madecassic acid, asiatic acid, oleanolic acid, and ursonic acid.

**In** one embodiment, the triterpenoid compound may be a combination of asiaticoside, madecassic acid, and asiatic acid.

**In** one embodiment, the phospholipid may be contained **in** an amount of 0.1 to 5 wt% relative to a total weight of the membrane components of the nanoliposome. Specifically, the phospholipid may be contained in an amount of 0.1 wt% or more, 0.5 wt% or more, 0.8 wt% or more, 0.9 wt% or more, 1 wt% or more, 1.2 wt% or more, 1.5 wt% or more, 2 wt% or more, 3 wt% or more, or 4 wt% or more relative to a total weight of the membrane components of the nanoliposome, and also, the phospholipid may be contained **in** an amount of 5 wt% or less, 4.5 wt% or less, 4 wt% or less, 3.5 wt% or less, 3 wt% or less, 2.5 wt% or less, 2 wt% or less, 1.5 wt% or less, 1.2 wt% or less, 1 wt% or less, or 0.9 wt% or less relative to a total weight of the membrane components of the nanoliposome.

**In** one embodiment, the phospholipid may be contained **in** an amount of 0.001 to 5 wt% relative to a total weight of the nanoliposome. Specifically, the phospholipid may be contained in an amount of 0.001 wt% or more, 0.005 wt% or more, 0.01 wt% or more, 0.05 wt% or more, 0.1 wt% or more, 0.5 wt% or more, 0.8 wt% or more, 0.9 wt% or more, 1 wt% or more, 1.2 wt% or more, 1.5 wt% or more, 2 wt% or more, 3 wt% or more, or 4 wt% or more relative to a total weight of the nanoliposome, and also, may be contained **in** an amount of 5 wt% or less, 4.5 wt% or less, 4 wt% or less, 3.5 wt% or less, 3 wt% or less, 2.5 wt% or less, 2 wt% or less, 1.5 wt% or less, 1.2 wt% or less, 1 wt% or less, 0.9 wt% or less, 0.8 wt% or less, 0.7 wt% or less, 0.6 wt% or less, 0.5 wt% or less, 0.4 wt% or less, 0.3 wt% or less, 0.2 wt% or less, 0.1 wt% or less, 0.05 wt% or less, 0.01 wt% or less, or 0.005 wt% or less.

**In** one embodiment, the phospholipid may include one or more selected from among a natural phospholipid, a semi-synthetic phospholipid, and a synthetic phospholipid.

**In** one embodiment, the natural phospholipid may be, for example, natural lecithin, soybean lecithin, egg yolk lecithin, or brain lecithin.

**In** one embodiment, the semi-synthetic phospholipid or synthetic phospholipid may be, for example, saturated phosphatidylcholine (PC) such as hydrogenated soybean phosphatidylcholine (HSPC), soybean phosphatidylcholine (SPC), dipalmitoyl phosphocholine (DPPC), distearoyl phosphocholine (DSPC), dioleoyl phosphocholine (DOPC), dilauroyl phosphocholine (DLPC), dierucoyl phosphocholine (DEPC), dimyristoyl phosphocholine (DMPC), palmitoyl oleoyl phosphocholine (POPC), stearoyl lysolecithin (S-LysoPC), myristoyl palmitoyl phosphocholine (MPPC), and glycerophosphocholine (GPC); phosphatidylglycerol (PG) such as distearoyl phospholipid (DSPG), palmitoyl phospholipid (DPPG), and dimyristoyl phospholipid (DMPG); phosphatidylinositol (PI); phosphatidylethanolamine (PE) such as distearoyl group phospholipid (DSPE), dioleoyl group phospholipid (DOPE), 1-palmitoyl-2-oleoyl phospholipid (POPE), dilauroyl phospholipid (DLPE), and dierucoyl phospholipid (DEPE); phosphatidylserine (PS); or a polyethylene glycolated phospholipid.

In one embodiment, the phospholipid may include one or more selected from among lysophosphatidylcholine, phosphatidic acid, and phosphatidylinositol.

In one embodiment, the phospholipid may be lecithin or hydrogenated lecithin.

In one embodiment, the cholesterol may be contained in an amount of 0.01 wt% or more and less than 3 wt% relative to a total weight of the membrane components of the nanoliposome. Specifically, the cholesterol may be contained in an amount of 0.01 wt% or more, 0.05 wt% or more, 0.1 wt% or more, 0.2 wt% or more, 0.3 wt% or more, 0.4 wt% or more, 0.5 wt% or more, 0.6 wt% or more, 0.7 wt% or more, 0.8 wt% or more, 1 wt% or more, or 2 wt% or more relative to a total weight of the membrane components of the nanoliposome, and also, the cholesterol may be contained in an amount of less than 3 wt%, 2.8 wt% or less, 2.6 wt% or less, 2.4 wt% or less, 2.2 wt% or less, 2 wt% or less, 1.8 wt% or less, 1.6 wt% or less, 1.4 wt% or less, 1.2 wt% or less, 1 wt% or less, 0.9 wt% or less, 0.8 wt% or less, 0.7 wt% or less, 0.6 wt% or less, or 0.1 wt% or less relative to a total weight of the membrane components of the nanoliposome.

In one embodiment, the cholesterol may be contained in an amount of 0.0001 to 5 wt% relative to a total weight of the nanoliposome. Specifically, the cholesterol may be contained in an amount of 0.0001 wt% or more, 0.0005 wt% or more, 0.001 wt% or more, 0.005 wt% or more, 0.01 wt% or more, 0.05 wt% or more, 0.1 wt% or more, 0.5 wt% or more, 0.8 wt% or more, 0.9 wt% or more, 1 wt% or more, 1.2 wt% or more, 1.5 wt% or more, 2 wt% or more, 3 wt% or more, or 4 wt% or more relative to a total weight of the nanoliposome, and also, may be contained in an amount of 5 wt% or less, 4.5 wt% or less, 4 wt% or less, 3.5 wt% or less, 3 wt% or less, 2.5 wt% or less, 2 wt% or less, 1.5 wt% or less, 1.2 wt% or less, 1 wt% or less, 0.9 wt% or less, 0.8 wt% or less, 0.7 wt% or less, 0.6 wt% or less, 0.5 wt% or less, 0.4 wt% or less, 0.3 wt% or less, 0.2 wt% or less, 0.1 wt% or less, 0.05 wt% or less, 0.01 wt% or less, or 0.005 wt% or less.

In one embodiment, the polyol may be contained in an amount of 70 to 98 wt% relative to a total weight of the membrane components of the nanoliposome. Specifically, the polyol may be contained in an amount of 70 wt% or more, 75 wt% or more, 80 wt% or more, 85 wt% or more, 86 wt% or more, 87 wt% or more, 88 wt% or more, 89 wt% or more, 90 wt% or more, 91 wt% or more, 92 wt% or more, 93 wt% or more, 94 wt% or more, 95 wt% or more, or 96 wt% or more relative to a total weight of the membrane components of the nanoliposome, and also, the polyol may be contained in an amount of 98 wt% or less, 97 wt% or less, 96 wt% or less, 95 wt% or less, 94 wt% or less, 93 wt% or less, 92 wt% or less, 91 wt% or less, or 90 wt% or less relative to a total weight of the membrane components of the nanoliposome.

In one embodiment, the polyol may be contained in an amount of 1 to 70 wt% relative to a total weight of the nanoliposome. Specifically, the polyol may be contained in an amount of 1 wt% or more, 5 wt% or more, 10 wt% or more, 15 wt% or more, 20 wt% or more, 25 wt% or more, 30 wt% or more, 35 wt% or more, 40 wt% or more, 45 wt% or more, 50 wt% or more, 55 wt% or more, 60 wt% or more, or 65 wt% or more relative to a total weight of the nanoliposome, and also, may be contained in an amount of 70 wt% or less, 65 wt% or less, 60 wt% or less, 55 wt% or less, 50 wt% or less, 45 wt% or less, 40 wt% or less, 35 wt% or less, 30 wt% or less, 25 wt% or less, 20 wt% or less, 15 wt% or less, 10 wt% or less, or 5 wt% or less.

In one embodiment, the polyol may be one or more selected from among glycerin, propylene glycol, butylene glycol, dipropylene glycol, tripropylene glycol, ethylene glycol, polyethylene glycol, pentylene glycol, propanediol, sorbitol, 1,2-hexanediol, isoprene glycol, and polyglycerin.

In one embodiment, the oil may be contained in an amount of 1 wt% or more and less than 10 wt% relative to a total weight of the membrane components of the nanoliposome. Specifically, the oil may be contained in an amount of 1 wt% or more, 1.5 wt% or more, 2 wt% or more, 2.5 wt% or more, 3 wt% or more, 3.5 wt% or more, 4 wt% or more, 4.1 wt% or more, 4.2 wt% or more, 4.3 wt% or more, 4.4 wt% or more, 4.5 wt% or more, 4.6 wt% or more, 4.7 wt% or more, 5 wt% or more, 5.5 wt% or more, 6 wt% or more, 7 wt% or more, or 8 wt% or more relative to a total weight of the membrane components of the nanoliposome, and also, the oil may be contained in an amount of less than 10 wt%, 9 wt% or less, 8 wt% or less, 7 wt% or less, 6.5 wt% or less, 6 wt% or less, 5.8 wt% or less, 5.6 wt% or less, 5.4 wt% or less, 5.2 wt% or less, 5 wt% or less, 4.9 wt% or less, 4.8 wt% or less, 4.7 wt% or less, 4.5 wt% or less, 4 wt% or less, or 3 wt% or less relative to a total weight of the membrane components of the nanoliposome.

In one embodiment, the oil may be contained in an amount of 0.001 to 10 wt% relative to a total weight of the nanoliposome. Specifically, the oil may be contained in an amount of 0.001 wt% or more, 0.005 wt% or more, 0.01 wt% or more, 0.05 wt% or more, 0.1 wt% or more, 0.5 wt% or more, 1 wt% or more, 1.5 wt% or more, 2 wt% or more, 2.5 wt% or more, 3 wt% or more, 3.5 wt% or more, 4 wt% or more, 4.5 wt% or more, 5 wt% or more, 5.5 wt% or more, 6 wt% or more, 7 wt% or more, 8 wt% or more, or 9 wt% or more relative to a total weight of the nanoliposome, and also, may be contained in an amount of 10 wt% or less, 9 wt% or less, 8 wt% or less, 7 wt% or less, 6.5 wt% or less, 6 wt% or less, 5.5 wt% or less, 5 wt% or less, 4.5 wt% or less, 4 wt% or less, 3.5 wt% or less, 3 wt% or less, 2.5 wt% or less, 2 wt% or less, 1.5 wt% or less, 1 wt% or less, 0.5 wt% or less, 0.1 wt% or less, 0.05 wt% or less, 0.01 wt% or less, or 0.005 wt% or less.

In one embodiment, the oil may be medium-chain triglycerides (MCT). Specifically, the oil may be one or more selected from among hydrogenated polyisobutene, hydrogenated polydecene, squalane, hydrogenated poly(C6-14 olefin), caprylic/capric triglyceride, cetyl ethylhexanoate, isopropyl myristate, isopropyl palmitate, ethylhexyl palmitate, diethylhexyl carbonate, isostearyl isostearate, sucrose stearate, cetyl octanoate, coco-caprylate/caprate, myristyl myristate, dicaprylyl carbonate, pentaerythrityl tetraisostearate, pentaerythrityl tetraethylhexanoate, diethoxyethyl succinate, dicaprylyl ether, 2-octyldodecanol, octyl-dodecyl myristate, and butylene glycol dicaprylate, and preferably may be caprylic/capric triglyceride.

In one embodiment, the triterpenoid compound may be contained in an amount of 0.05 wt% or more and less than 2 wt% relative to a total weight of the membrane components of the nanoliposome. Specifically, the triterpenoid compound may be contained in an amount of 0.05 wt% or more, 0.1 wt% or more, 0.2 wt% or more, 0.3 wt% or more, 0.4 wt% or more, 0.5 wt% or more, 1 wt% or more, or 1.5 wt% or more relative to a total weight of the membrane components of the nanoliposome, and also, the triterpenoid compound may be contained in an amount of less than 2 wt%, 1.8 wt% or less, 1.6 wt% or less, 1.4 wt% or less, 1.2 wt% or less, 1 wt% or less, 0.9 wt% or less, 0.8 wt% or less, 0.7 wt% or less, 0.6 wt% or less, 0.5 wt% or less, 0.3 wt% or less, or 0.1 wt% or less relative to a total weight of the membrane components of the nanoliposome.

In one embodiment, the triterpenoid compound may be contained in an amount of 0.001 to 5 wt% relative to a total weight of the nanoliposome. Specifically, the triterpenoid compound may be contained in an amount of 0.001 wt% or more, 0.005 wt% or more, 0.01 wt% or more, 0.05 wt% or more, 0.1 wt% or more, 0.5 wt% or more, 0.8 wt% or more, 0.9 wt% or more, 1 wt% or more, 1.2 wt% or more, 1.5 wt% or more, 2 wt% or more, 3 wt% or more, or 4 wt% or more relative to a total weight of the nanoliposome, and also, may be contained in an amount of 5 wt% or less, 4.5 wt% or less, 4 wt% or less, 3.5 wt% or less, 3 wt% or less, 2.5 wt% or less, 2 wt% or less, 1.5 wt% or less, 1.2 wt% or less, 1 wt% or less, 0.9 wt% or less, 0.8 wt% or less, 0.7 wt% or less, 0.6 wt% or less, 0.5 wt% or less, 0.4 wt% or less, 0.3 wt% or less, 0.2 wt% or less, 0.1 wt% or less, 0.05 wt% or less, 0.01 wt% or less, or 0.005 wt% or less.

In one embodiment, the nanoliposome may load an efficacy ingredient.

In one embodiment, the efficacy ingredient may be a fat-soluble efficacy ingredient such as Melasolv, phytosterol, ceramide, retinol, and Compound K; or a water-soluble efficacy ingredient such as caffeine, acetaminophen, antipyrine, theophylline, riboflavin, PDRN, vitamin C, and niacinamide.

In one embodiment, the fat-soluble efficacy ingredient may be encapsulated in the nanoliposome by being dissolved in the oil or polyol.

In one embodiment, the water-soluble efficacy ingredient may be loaded in the nanoliposome by being input together with water during manufacturing of the nanoliposome, or by being dissolved and mixed in the polyol. The present invention, in another aspect, may relate to a cosmetic composition including the nanoliposome.

In one embodiment, the cosmetic composition may include the nanoliposome; and an efficacy ingredient loaded in the nanoliposome.

In one embodiment, the cosmetic composition may be manufactured in various formulations, and may be implemented, for example, in formulations such as an essence, serum, cream, lotion, gel, spray, mist, oil, foam, mask pack, ampoule, cleanser, and makeup products (e.g., foundation, primer, etc.).

In one embodiment, the cosmetic composition may additionally include additives commonly used in a cosmetic field besides the above described. For example, a pH adjusting agent (e.g., citric acid, tromethamine), a moisturizer (e.g., glycerin, butylene glycol), an emulsifier, a surfactant, a viscosity adjusting agent (e.g., carbomer), a fragrance, a preservative, a pigment, and an antioxidant (e.g., tocopherol, ascorbyl palmitate, butylhydroxytoluene (BHT)), etc. may be added.

The additional components may be appropriately selected and blended by those skilled in the art by considering functionality and safety in order to improve stability, feeling of use, preservation power, etc. of the composition, and those skilled in the art may select any additional component and/or an amount thereof such that advantageous characteristics of the nanoliposome according to the present specification are not adversely affected or are not substantially affected by addition.

The present invention, in still another aspect, may relate to a manufacturing method of the nanoliposome.

In one embodiment, the manufacturing method may include a first mixing step of mixing a phospholipid, cholesterol, a polyol, and a triterpenoid compound; and a second mixing step of inputting water into the mixture and mixing.

In one embodiment, an oil may be additionally mixed in the first mixing step.

Since specific details regarding the nanoliposome, phospholipid, cholesterol, polyol, oil, and triterpenoid compound are as described in detail above, omission is made below.

In one embodiment, a mixing temperature in the first mixing step may be 25 to 110°C. Specifically, a mixing temperature in the first mixing step may be 25°C or more, 30°C or more, 35°C or more, 40°C or more, 45°C or more, 50°C or more, 60°C or more, 70°C or more, 80°C or more, 90°C or more, 100°C or more, or 105°C or more. Also, a mixing temperature in the first mixing step may be 110°C or less, 105°C or less, 100°C or less, 95°C or less, 90°C or less, 85°C or less, 80°C or less, 75°C or less, 70°C or less, 65°C or less, 60°C or less, 55°C or less, 50°C or less, 45°C or less, 40°C or less, 35°C or less, or 30°C or less.

In one embodiment, a temperature of the water may be 25 to 110°C. Specifically, a temperature of the water may be 25°C or more, 30°C or more, 35°C or more, 40°C or more, 45°C or more, 50°C or more, 60°C or more, 70°C or more, 80°C or more, 90°C or more, 100°C or more, or 105°C or more. Also, a temperature of water may be 110°C or less, 105°C or less, 100°C or less, 95°C or less, 90°C or less, 85°C or less, 80°C or less, 75°C or less, 70°C or less, 65°C or less, 60°C or less, 55°C or less, 50°C or less, 45°C or less, 40°C or less, 35°C or less, or 30°C or less.

In one embodiment, in the second mixing step, the water may be input in an amount of 1 to 5 times relative to a total weight of the mixture. Specifically, in the second mixing step, the water may be input in an amount of 1 time or more, 1.5 times or more, 2 times or more, 2.5 times or more, 3 times or more, 3.1 times or more, 3.2 times or more, 3.3 times or more, 3.4 times or more, 3.5 times or more, 3.6 times or more, 3.65 times or more, 3.7 times or more, 3.8 times or more, 3.9 times or more, or 4 times or more relative to a total weight of the mixture, and also, the water may be input in an amount of 5 times or less, 4.9 times or less, 4.8 times or less, 4.7 times or less, 4.6 times or less, 4.5 times or less, 4.4 times or less, 4.3 times or less, 4.2 times or less, 4.1 times or less, 4 times or less, 3.9 times or less, 3.8 times or less, 3.7 times or less, 3.6 times or less, 3.5 times or less, 3.4 times or less, 3.3 times or less, 3.2 times or less, 3.1 times or less, 3 times or less, 2 times or less, or 1.5 times or less relative to a total weight of the mixture.

In one embodiment, in the first mixing step, the polyol may dissolve the phospholipid, the cholesterol, and the triterpenoid compound to form a multilamellar giant vesicle.

In one embodiment, in the second mixing step, the multilamellar giant vesicle formed in the first mixing step may be fragmented into a unilamellar nanoliposome by an osmotic pressure gradient due to mixing with water.

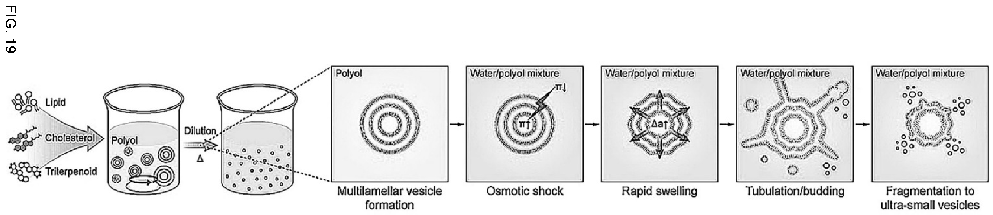

In one embodiment, the nanoliposome may be formed by a polyol-based osmotic pressure-driven fragmentation mechanism (FIG. 19). Specifically, in the manufacturing method of the nanoliposome, a polyol acts as a solvent dissolving a phospholipid, cholesterol, and a triterpenoid compound, thereby providing an environment in which lipids are spontaneously assembled into a multilamellar giant vesicle. Afterward, when inputting water and diluting, a rapid osmotic pressure gradient reaching about 30 MPa is formed due to a membrane permeability difference between water molecules and polyol molecules, and while water molecules are rapidly introduced into an inside of a vesicle, large-sized polyol molecules are maintained in a state of being captured within the inside of the vesicle. Due to this, as the vesicle rapidly expands, an internal pressure exceeds a mechanical tolerance limit of a membrane, and eventually, the giant vesicle is fragmented in a controlled manner, so that a small unilamellar nanoliposome is formed. Such an osmotic pressure-driven fragmentation mechanism can manufacture a nanoliposome having a uniform particle distribution through a thermodynamically advantageous path compared to high-energy mechanical methods such as ultrasonication, extrusion, and high-pressure homogenization, and thus mass production may be possible while minimizing quality deviation between batches without structural damage.

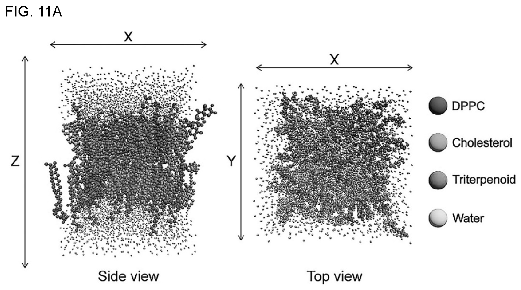

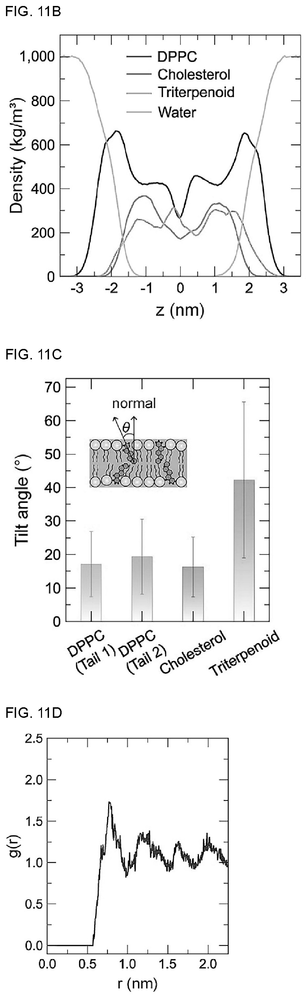

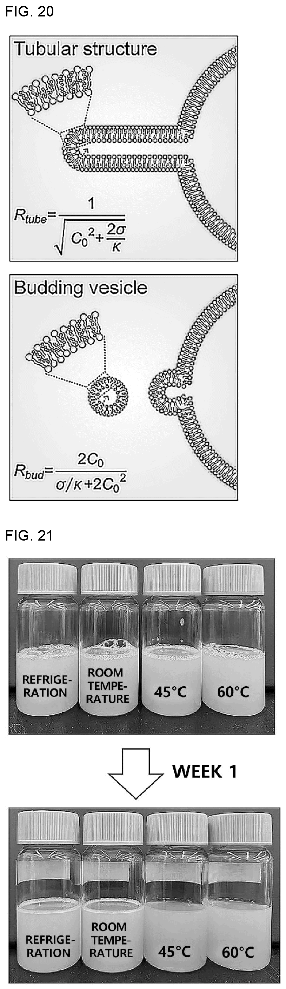

In one embodiment, an ultra-small particle size (less than 30 nm) of the nanoliposome may be achieved by a curvature induction mechanism of a triterpenoid compound. As shown in FIG. 20, according to a curvature-based theoretical framework, a condition that commonly satisfies a vesicle radius of 10 nm in both tubular structure and budding vesicle forms is met only when a spontaneous curvature C₀ = 0.2 nm⁻¹, and from this, it is established regardless of bending rigidity (κ) and membrane tension (σ), so it can be seen that the spontaneous curvature induced by a triterpenoid compound is a key factor governing size determination of the nanoliposome. Specifically, the triterpenoid compound is arranged with a large tilt angle of about 40° within a lipid bilayer to form a conical molecular geometry, which acts as a molecular driver inducing a positive spontaneous curvature similarly to classical curvature-generating proteins or lipids. Also, a triterpenoid compound is asymmetrically distributed in an outer leaflet of the bilayer and thus may form bilayer asymmetry, through which an energetically advantageous condition necessary for formation of an ultra-small vesicle having a diameter of less than 30 nm may be met (FIGS. 11A to 11D).

In one embodiment, improved membrane stability of the nanoliposome may be attributed to a hydrogen bonding network that a triterpenoid compound forms within a lipid bilayer. Specifically, a hydroxyl group of a triterpenoid compound is oriented toward an aqueous phase direction, whereas a carboxyl group participates in hydrogen bonding within a hydrophobic core inside a membrane, and accordingly, extensive hydrogen bonding is formed between triterpenoid molecules and between triterpenoid and lipid molecules. By such a physical cross-linking effect, the nanoliposome may exhibit excellent resistance against severe external conditions such as high temperature, extreme pH, and surfactant treatment despite being an ultra-small nanoliposome.

The present invention, as one embodiment, may provide the following embodiments.

A first embodiment may provide a nanoliposome, including, as membrane components of the nanoliposome, a phospholipid; cholesterol; a polyol; and a triterpenoid compound.

A second embodiment may provide the nanoliposome according to the first embodiment, wherein the nanoliposome further includes an oil as a membrane component.

A third embodiment may provide the nanoliposome according to one or more of the first embodiment and the second embodiment, wherein a particle size of the nanoliposome is less than 30 nm.

A fourth embodiment may provide the nanoliposome according to one or more of the first embodiment to the third embodiment, wherein the particle size of the nanoliposome is 15 nm or more and less than 30 nm.

A fifth embodiment may provide the nanoliposome according to one or more of the first embodiment to the fourth embodiment, wherein the triterpenoid compound is one or more selected from among asiaticoside, madecassic acid, asiatic acid, oleanolic acid, and ursonic acid.

A sixth embodiment may provide the nanoliposome according to one or more of the first embodiment to the fifth embodiment, wherein the triterpenoid compound is a combination of asiaticoside, madecassic acid, and asiatic acid.

A seventh embodiment may provide the nanoliposome according to one or more of the first embodiment to the sixth embodiment, wherein the phospholipid is contained in an amount of 0.001 to 5 wt% relative to a total weight of the nanoliposome.

An eighth embodiment may provide the nanoliposome according to one or more of the first embodiment to the seventh embodiment, wherein the cholesterol is contained in an amount of 0.0001 to 5 wt% relative to a total weight of the nanoliposome.

A ninth embodiment may provide the nanoliposome according to one or more of the first embodiment to the eighth embodiment, wherein the polyol is contained in an amount of 1 to 70 wt% relative to a total weight of the nanoliposome.

A tenth embodiment may provide the nanoliposome according to one or more of the second embodiment to the ninth embodiment, wherein the oil is contained in an amount of 0.001 to 10 wt% relative to a total weight of the nanoliposome.

An eleventh embodiment may provide the nanoliposome according to one or more of the first embodiment to the tenth embodiment, wherein the triterpenoid compound is contained in an amount of 0.001 to 5 wt% relative to a total weight of the nanoliposome.

A twelfth embodiment may provide the nanoliposome according to one or more of the first embodiment to the eleventh embodiment, wherein the nanoliposome loads one or more efficacy ingredients selected from among caffeine, acetaminophen, antipyrine, theophylline, niacinamide, riboflavin, PDRN, vitamin C, Melasolv, Compound K, phytosterol, ceramide, and retinol.

A thirteenth embodiment may provide a cosmetic composition including the nanoliposome according to one or more of the first embodiment to the twelfth embodiment.

A fourteenth embodiment may provide a manufacturing method of the nanoliposome according to one or more of the first embodiment to the twelfth embodiment, including: a first mixing step of mixing a phospholipid, cholesterol, a polyol, and a triterpenoid compound; and a second mixing step of mixing the mixture with water.

A fifteenth embodiment may provide the manufacturing method of the nanoliposome according to the fourteenth embodiment, wherein, in the first mixing step, an oil is additionally mixed.

A sixteenth embodiment may provide the manufacturing method of the nanoliposome according to one or more of the fourteenth embodiment and the fifteenth embodiment, wherein a mixing temperature in the first mixing step is 25 to 110°C.

A seventeenth embodiment may provide the manufacturing method of the nanoliposome according to one or more of the fourteenth embodiment to the sixteenth embodiment, wherein a temperature of the water is 50 to 110°C.

An eighteenth embodiment may provide the manufacturing method of the nanoliposome according to one or more of the fourteenth embodiment to the seventeenth embodiment, wherein, in the first mixing step, the polyol may dissolve the phospholipid, the cholesterol, and the triterpenoid compound to form a multilamellar giant vesicle.

A nineteenth embodiment may provide the manufacturing method of the nanoliposome according to one or more of the fourteenth embodiment to the eighteenth embodiment, wherein, in the second mixing step, the multilamellar giant vesicle formed in the first mixing step is fragmented into a unilamellar nanoliposome by an osmotic pressure gradient due to mixing with water.

Hereinafter, contents of the present invention will be described more specifically through examples and test examples. However, such examples and test examples are only presented to understand the contents of the present invention, and a scope of rights of the present invention is not limited to such examples and test examples, and modifications, substitutions, insertions, and the like commonly known in the art may be performed, and these are also included in the scope of the present invention.

### [Manufacturing Example 1] Manufacture of general liposome and high-stability nanoliposome

Nanoliposomes (SFL, Examples 1 to 5) according to one embodiment of the present invention, an oil in water (O/W) formulation (Comparative Example 1), and general liposomes (G-Lip, Comparative Examples 2 to 7) were manufactured with ingredients and contents according to Table 1 below. Specifically, after mixing ingredients of a polyol part at a temperature of 85 to 95°C, distilled water of 85 to 95°C was mixed, and Comparative Examples 1 to 7 and Examples 1 to 5 were manufactured by mixing for 5 minutes using a T.K Homomixer Mark2 mixer (PRIMIX). In this case, as a triterpenoid compound, oleanolic acid, or CENTELLA ASIATICA TRITERP (obtained from Givaudan), which is a complex of madecassic acid, asiatic acid, and asiaticoside, was used.

**TABLE 1**

| | | Comparative Example | | | | | | | Example | | | | | |
|---|---|---|---|---|---|---|---|---|---|---|---|---|---|---|
| | | 1 (O/ W) | 2 (G-Lip) | 3 | 4 | 5 | 6 | 7 | | 1 (SF L-1) | 2 (SF L-2) | 3 (SF L-3) | 4 (UF N-Lip) | 5 (UF N-Lip) |
| Polyol Part | Caprylic/ Capric Triglyceride | 1 | 1 | - | 2.3 | 1 | 1 | 1 | | 1 | 1 | 1 | - | 1 |
| | Cholesterol | 0.13 | 0.13 | 0.13 | 0.13 | - | 0.66 | 0.13 | | 0.13 | 0.13 | 0.13 | 0.1 | 0.1 |
| | Lecithin | 0.25 | 0.25 | 0.25 | 0.25 | 0.25 | 0.25 | 0.25 | | 0.25 | 0.25 | 0.25 | 0.25 | 0.25 |
| | 1,3-Butylene Glycol | 10 | 10 | - | 10 | 10 | 10 | 10 | | 10 | 10 | 10 | - | - |
| | Glycerol | 10 | 10 | - | 10 | 10 | 10 | 10 | | 10 | 10 | 10 | - | - |
| | Propylene Glycol | - | - | 30 | - | - | - | - | | - | - | - | 30 | 30 |
| | Oleanolic | - | - | - | - | - | - | 0.44 | | 0.1 | - | - | | - |
| | Acid | | | | | | | | | | | | | |
| | Madecassic Acid | - | - | - | - | - | | - | - | - | - | - | 0.1 | 0.1 |
| | CENTELLA ASIATICA TRITERP | - | - | - | - | - | | - | - | - | 0.1 | 0.3 | - | - |
| Distilled Water | | To 100 | To 100 | To 100 | To 100 | To 100 | | To 100 | To 100 | To 100 | To 100 | To 100 | To 100 | To 100 |

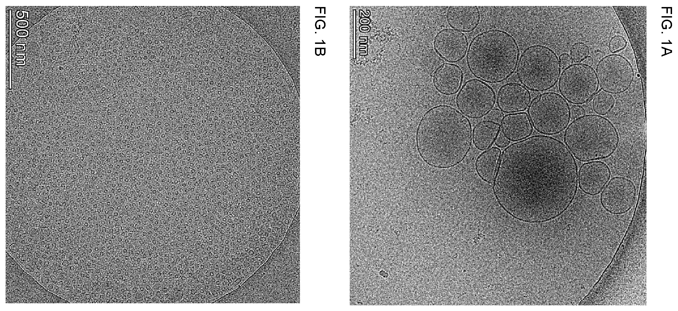

Results of photographing particle sizes of manufactured nanoliposomes of Comparative Example 3 and Example 4 using cryogenic transmission electron microscopy (Cryo-TEM) are shown in FIGS. 1A and 1B.

As shown in FIGS. 1A and 1B, it was confirmed that, in a case of a general liposome according to Comparative Example 3, it has a very large particle size of 100 to 300 nm, whereas in a case of the nanoliposome of Example 4 according to one embodiment of the present invention, it has a very small particle size at a level of 15 to 28 nm.

### [Test Example 1] Skin stratum corneum permeability confirmation test

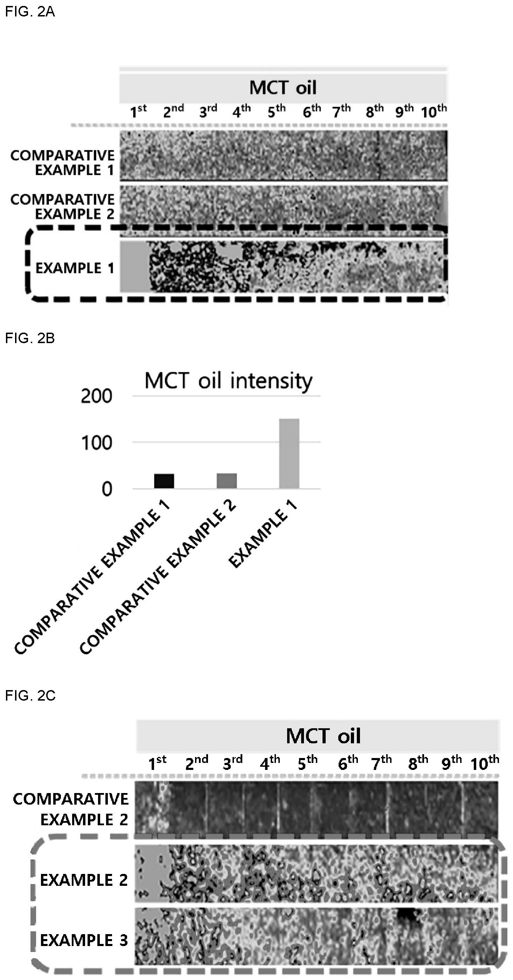

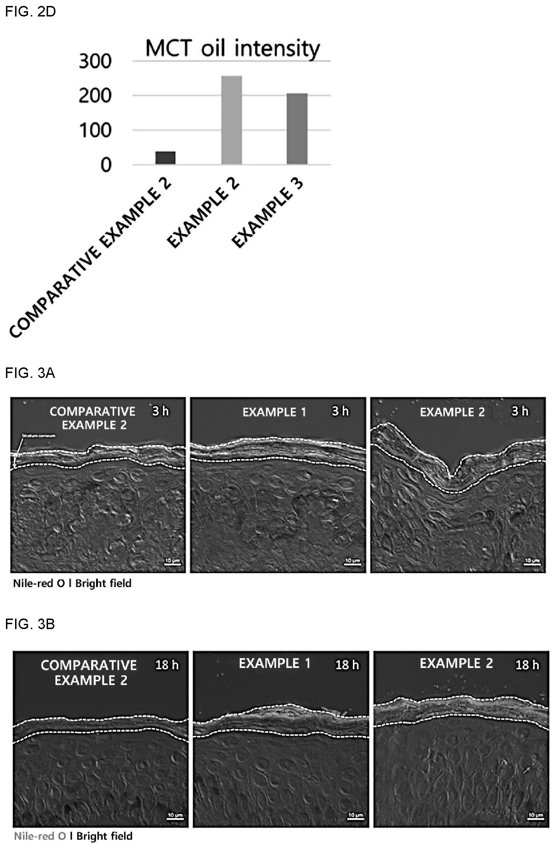

Skin stratum corneum permeability was confirmed using a Franz diffusion cell (Microetteplus, Teledyne Hanson, Los Angeles, CA, USA). Specifically, a receptor chamber was filled with a PBS solution, and a temperature was maintained at 32 ± 0.5°C while stirring at 600 rpm. Excised full-thickness skin specimens (obtained from HansBioMed) were fixed between a donor chamber and the receptor chamber, and in this case, an area in contact with a receptor phase was about 1.767 cm². Afterward, after applying 0.5 g each of Comparative Examples 1 and 2, and Examples 1 to 3 manufactured in the above Manufacturing Example to skin specimens, after 4 hours, the skin specimens were separated from the Franz diffusion cell, wiped with a cotton swab, and washed with distilled water to remove test substances remaining on a skin surface. Next, after attaching the skin specimens onto Parafilm fixed to a solid surface, they were dried by tapping with Kimwipes, and D-Squame tape (D100, CuDerm) was attached to the skin specimens. Pressure was evenly applied to the tape using a pressure instrument (D-Squame Pressure Instrument, CuDerm). After applying pressure for 5 seconds, the tape was quickly peeled off using tweezers, and afterward, 11 layers were collected by attaching and stripping D-Squame tape (D100, CuDerm), then the first tape strip was discarded, and DESI-MSI analysis was performed on the remaining 10 layers. The results are shown in FIGS. 2A to 2D.

From the results of FIGS. 2A to 2D, it was confirmed that, while Comparative Examples 1 and 2 hardly pass through a skin stratum corneum, in a case of nanoliposomes of Examples 1 to 3, oil is well delivered up to a 10th layer of the stratum corneum.

### [Test Example 2] Ex-vivo skin permeability confirmation test for skin biopsy tissue

Ex-vivo imaging was performed by encapsulating fluorescence-labeled oil (MCT oil) in the general liposome of Comparative Example 2 and nanoliposomes of Examples 1 and 2, to confirm how much deeper and how much faster the liposomes permeate. Specifically, Comparative Example 2, Example 1, and Example 2 were applied onto skin biopsy tissue (abdominal skin of a 54-year-old female purchased from Genoskin Co., #NSA20) for 3 to 18 hours, and imaging was performed using a confocal microscope, and results thereof are shown in FIGS. 3A to 3D.

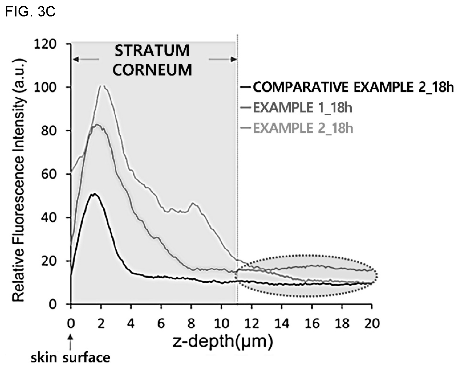

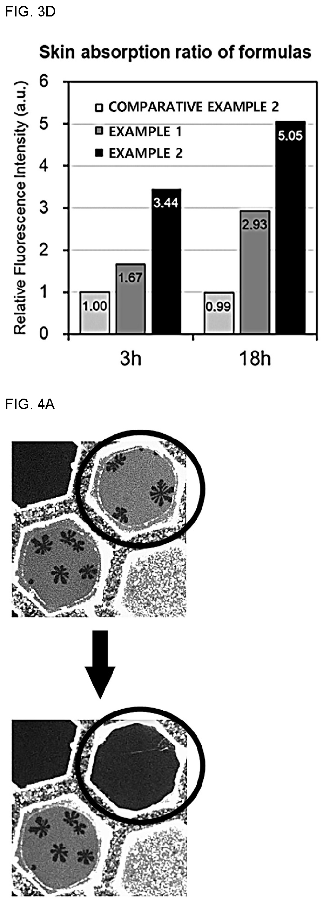

As shown in FIG. 3A (measured after 3 hours of loading), FIG. 3B (measured after 18 hours of loading), FIG. 3C (graph of relative fluorescence signal intensity according to depth (z-axis)), and FIG. 3D (fluorescence signal intensity per specimen), it was confirmed that, while the general liposome according to Comparative Example 2 exists in a shallow stratum corneum, the nanoliposomes of Examples 1 and 2 permeated 2 to 4 times or more deeper compared to the general liposome, and an amount thereof also permeated the skin in an amount of 3.5 to 5 times or more greater compared to the general liposome.

### [Test Example 3] Membrane structure stability confirmation test

To confirm stability of a bilayer constituting a liposome, a free-standing lipid bilayer membrane was manufactured using each of the general liposome according to Comparative Example 3 and the nanoliposome according to Example 1, and then a membrane breaking form under an unstable environment (stress) was observed. Specifically, first, lipids (polyol part ingredients of Comparative Example 3 and Example 1) were dissolved in chloroform in contents of Table 1 and mixed in a glass vial, then dried using gentle nitrogen (N₂) gas. The dried lipids were dissolved in a mixture of hexadecane and silicone oil (1:1 v/v) to make a lipid oil solution having a total lipid concentration of 5 mM. Each oil was filtered with a 0.2 µm syringe filter (Whatman) to remove impurities before use, and the lipid oil solution was ultrasonicated for 1 hour. An aqueous buffer solution including a total of 1 mL of PLL-PEG was injected into an imaging chamber, and 2 µL of the lipid oil solution was carefully dropped and spread on an air-aqueous buffer solution interface, then about 3 minutes after dropping the lipid oil solution, a hexagonal TEM grid (G200HEX, G150HEX, G100HEX, Gilder Grids) treated with 1-dodecanethiol for hydrophobic coating was carefully placed on an air-oil interface with tweezers. Afterward, for 2 minutes, a hydrophobic surface of the TEM grid was wetted by the lipid oil solution, and holes of the grid were filled with a thin oil film. This grid was submerged into the aqueous buffer solution using a syringe needle and placed on a glass surface coated with PLL-PEG. As oil was discharged, an oil film thickness became sufficiently thin so that two lipid monolayers were joined, and as a result, a planar free-standing lipid bilayer membrane is formed.

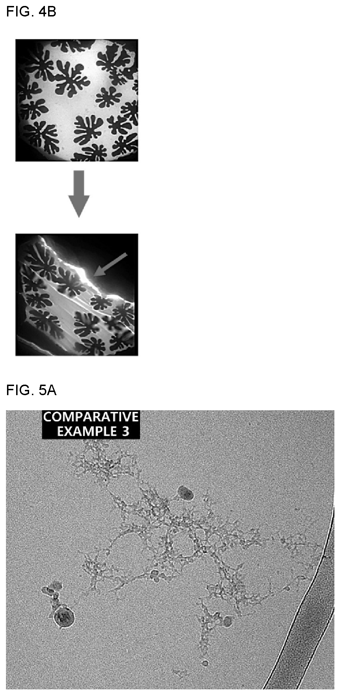

A lipid membrane array was cooled to 24°C after incubation at 45°C for at least 30 minutes, and after incubation of about 1 hour, a prepared OA (oleanolic acid)-MβCD (10 mM, 20:1 mol%) solution was injected into the aqueous buffer solution at a desired concentration. An unstable environment was created by applying pressure using a pipette, and all experiments were performed at 24°C, and the lipid membrane was directly visualized using an Inverted Microscope (IX73, Olympus) and an iXon EMCCD camera (X-6880, Andor Solis), and images were analyzed using an open-source program Fiji. The results are shown in FIGS. 4A and 4B.

As shown in FIGS. 4A and 4B, it was confirmed that, in a case of a bilayer membrane structure made of general liposome constituent materials according to Comparative Example 3, a membrane bursts according to a change in an external environment, whereas in a case of a bilayer membrane structure made of nanoliposome constituent materials according to Example 4, only one side is torn while a form of a membrane is maintained. From this, it was found that in the nanoliposome according to Example 1, membrane strength is still maintained even under a change in an external environment.

### [Test Example 4] Membrane structure stability confirmation test under severe conditions

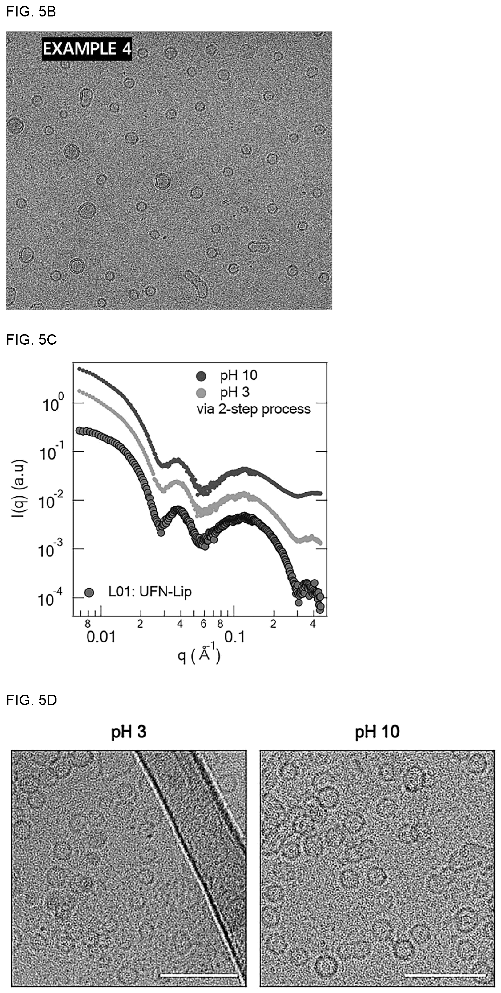

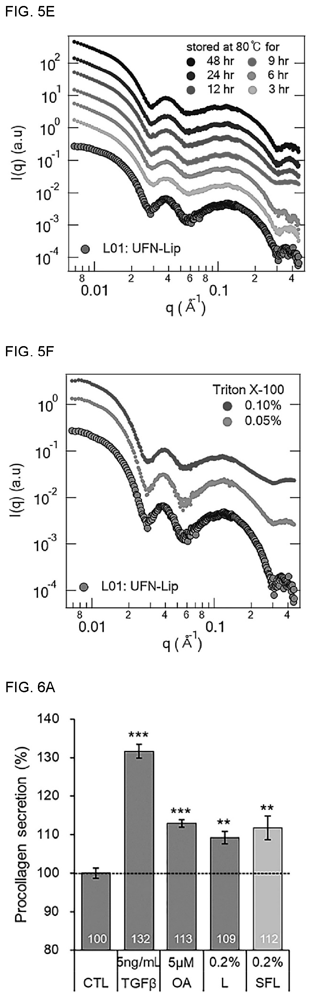

Regarding the general liposome of Comparative Example 3 and the nanoliposome of Example 4, stability of a membrane structure was confirmed under severe conditions of temperature, pH, and surfactant treatment. Specifically, a change in a membrane structure was confirmed through particle size measurement (SAXS) (using Pohang Light Source II PLS-II 4C SAXS II beamline, X-ray wavelength 0.734 Å, Si(111) double-crystal monochromator, detector-specimen distance 0.5 m and 4 m, q range 0.006 Å⁻¹ < q < 0.5 Å⁻¹, 1.5 mm diameter quartz capillary cell, and EIGER2 X 4M hybrid photon counting detector for 2D scattering data collection followed by radial averaging and absolute scale conversion) and cryogenic transmission electron microscopy (Cryo-TEM) (Quantifoil R2/2 Au 300 mesh grid or Lacey Carbon 200 mesh grid, glow discharge 30-50 seconds, 10-22 mA, specimen 2-5 µL loading, Vitrobot Mark IV, vitrification under 4-8°C and >95% relative humidity conditions, and 120 kV or 300 kV acceleration voltage) while storing under a high-temperature condition at 80°C for 48 hours. In a case of pH, a change in a membrane structure at pH 3 and pH 10 was confirmed through particle size measurement (SAXS) and Cryo-TEM, and a change in a membrane structure by treatment per concentration (0.1% w/v, 0.05% w/v) of a surfactant (Triton X-100) was confirmed through particle size measurement (SAXS). The results are shown in FIGS. 5A to 5F.

From the results of FIGS. 5A and 5B, it was confirmed that, in a case of the general liposome of Comparative Example 3, after 48 hours at high temperature, liposomes almost disappear and an aggregation phenomenon occurs so that turbidity increases, whereas in a case of the nanoliposome of Example 4, liposomes are maintained as they are even after 48 hours at high temperature.

Also, from the results of FIGS. 5C to 5F, it was confirmed that, in the nanoliposome (UFN-Lip) of Example 4, there is no change in a peak position of liposome particles even under low pH and high pH conditions, a high-temperature condition, and treatment of a surfactant that acts to break a membrane structure of a liposome, and a particle form and size are maintained in cryo-TEM images, so that a liposome membrane structure is maintained as it is.

### [Test Example 5] Cell efficacy confirmation test according to efficacy ingredient release

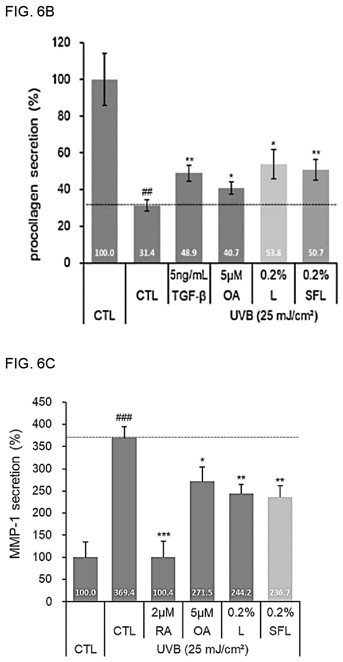

Cell efficacy according to release of an efficacy ingredient was confirmed by encapsulating oleanolic acid (OA) in the nanoliposome (SFL) of Example 1 manufactured in the above Manufacturing Example. Specifically, pure oleanolic acid (OA), OA (L) encapsulated in the general liposome of Comparative Example 2, and OA (SFL) encapsulated in the nanoliposome of Example 1 were treated to fibroblasts (human dermal fibroblast) per concentration (5 µM, 0.2% w/v, 0.2% w/v) for 2 days, respectively. In this case, OA was encapsulated by being dissolved in oil (caprylic/capric triglyceride), and a final concentration of OA contained in each specimen was adjusted to be a 5 µM concentration. A cell culture medium was collected, and Type I procollagen ELISA (R&D systems, #DY6220-05) was performed. In a case of ultraviolet stimulation conditions, immediately after irradiating 25 mJ/cm² UVB to fibroblasts, OA, L, and SFL were respectively put into a cell culture medium per concentration and cultured for 2 days. Then, the cell culture medium was collected, and Type I procollagen ELISA and MMP-1 ELISA (R&D systems, #DY901B) were performed. TGF-β (tumor growth factor-beta), well known as a procollagen secretion **promoting** ingredient, was used as a positive control, and RA (retinoic acid), well known as an MMP-1 secretion inhibiting ingredient, was used as a positive control, respectively. The results are shown in FIGS. 6A to 6C.

From the results of FIGS. 6A to 6C, it was confirmed that the nanoliposome according to one embodiment of the present invention exhibits cell efficacy by encapsulating and releasing an efficacy ingredient at a level similar to pure oleanolic acid and a general liposome.

### [Test Example 6] Formulation stability confirmation test

Regarding liposome formulations of Comparative Examples 1, 2, 4 to 7 and Examples 1 to 5 of Table 1, formulation stability upon lapse of 4 weeks was confirmed by visual observation, and results thereof are shown in Table 2 below. (Very excellent: ⊚, Excellent: ∘, Poor: Δ, Very poor: X)

**TABLE 2**

| | Formulation Stability (After 4 Weeks) |
|---|---|
| Comparative Example 1 | ○ |
| Comparative Example 2 | ⊚ |
| Comparative Example 4 | X |
| Comparative Example 5 | Δ |
| Comparative Example 6 | X |
| Comparative Example 7 | X |
| Example 1 | ⊚ |
| Example 2 | ⊚ |
| Example 3 | ⊚ |
| Example 4 | ⊚ |
| Example 5 | ⊚ |

From the results of Table 2, it was confirmed that, in cases of Comparative Example 4 including oil in about 10 wt% relative to a total weight of membrane components, Comparative Examples 5 and 6 not including cholesterol or including the same in about 3 wt%, and Comparative Example 7 including a triterpenoid compound in about 2 wt%, formulation stability is significantly lowered.

### [Test Example 7] Structural transition confirmation test of nanoliposome according to amount of water added

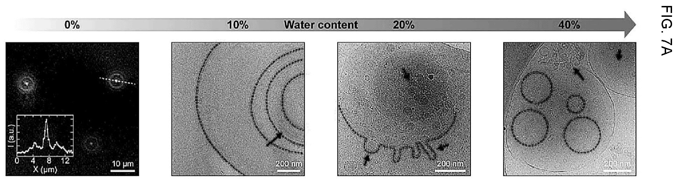

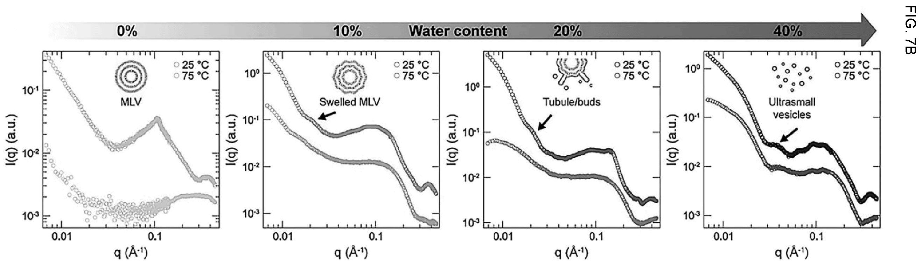

Using the nanoliposome (UFN-Lip) of Example 4 manufactured in the above Manufacturing Example, a membrane structural transition according to an amount of water added was confirmed through SAXS and cryo-TEM. Specifically, water was stepwise added to a triterpenoid compound-containing lipid mixture (polyol part) so that a water content became 0 wt%, 10 wt%, 20 wt%, and 40 wt%. A structural change at each water content was measured by SAXS under 25°C and 75°C conditions, and intermediate structures (tubes, budding vesicles, etc.) were observed by cryo-TEM. SAXS measurement and cryo-TEM observation conditions are the same as in Test Example 4. The results are shown in FIG. 7A (cryo-TEM) and FIG. 7B (SAXS).

From the results of FIGS. 7A and 7B, it was confirmed that, at 25°C, when a water content becomes 10 wt% or more, a characteristic scattering peak of 20 nm begins to appear, and at 20 wt%, intermediate structures of tubulation and budding were observed by cryo-TEM, and at 40 wt% or more, complete fragmentation into nanoliposomes was confirmed. On the other hand, at high temperature (75°C), the scattering peak of 20 nm does not clearly appear even at a high water content, and from this, it was confirmed that formation of nanoliposomes is inhibited.

### [Test Example 8] Nanostructure change confirmation test of nanoliposome according to water content

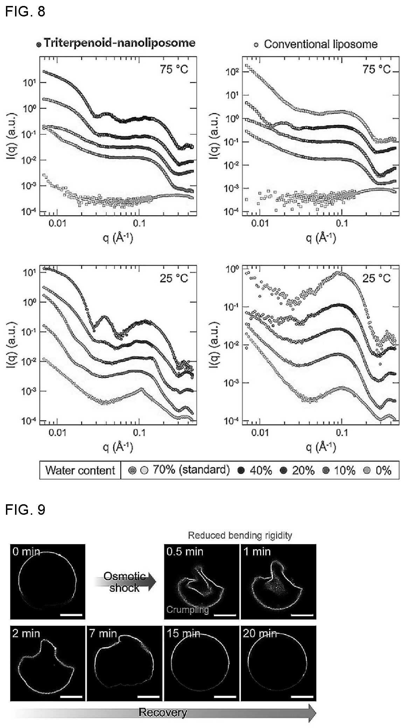

Regarding the nanoliposome (triterpenoid-nanoliposome) of Example 4 and the general liposome (conventional liposome) of Comparative Example 3 manufactured in the above Manufacturing Example, a nanostructure change according to water content was further confirmed through SAXS. Specifically, while stepwise adding water from 0 wt% to 70 wt% to a polyol part, a SAXS profile change was measured under the same conditions as in Test Example 4. The results are shown in FIG. 8.

From the results of FIG. 8, it was confirmed that, in a case of Example 4 containing a triterpenoid compound, a structural transition is gradually induced as a water content increases, which appears as a phenomenon in which a distinct scattering peak appears near q ≈ 0.03 Å⁻¹ and gradually becomes clear. Such results are consistent with the formation of nanoliposomes. On the other hand, in a case of Comparative Example 3 not including a triterpenoid compound, almost no change was observed within a measured q range, which suggests that there is no detectable structural rearrangement at a length scale of 100 nm or less.

### [Test Example 9] Morphology change confirmation test of triterpenoid-containing GUV (Giant Unilamellar Vesicle) under osmotic pressure conditions

To confirm an effect of a triterpenoid compound on bending rigidity of a lipid membrane, a DOPC-based model membrane system, GUV, was manufactured and a morphology change under osmotic pressure conditions was observed.

Specifically, 1,2-dioleoyl-sn-glycero-3-phosphocholine (DOPC) and 2 mol% biotin-cap-PE were dissolved in chloroform to manufacture a final concentration of 1 mg mL⁻¹. A lipid solution was applied to an ITO-coated glass slide (30 Ω, Omniscience) and dried under vacuum for 2 hours or more. A GUV was manufactured by an electroformation method, and under a condition of 55°C in a 200 mOsm/L glucose solution, a voltage was increased from 50 mVpp to 1400 mVpp at 10 Hz for 30 minutes, maintained at 1400 mVpp for 120 minutes, and then treated at 2200 mVpp at 5 Hz for 30 minutes. The manufactured GUV was fixed to a glass coverslip by biotin-streptavidin interaction using biotin-BSA (1 mg mL⁻¹, 30-minute incubation) and streptavidin (0.2 mg mL⁻¹, 30-minute incubation).

For insertion of a triterpenoid compound into a GUV membrane, oleanolic acid (9.1 mg) was added to 10 mL of an aqueous buffer solution (100 mM NaCl, 10 mM HEPES, pH 7.4) containing 40 mM MβCD (methyl-β-cyclodextrin) and mixed at a molar ratio of 1:20 (oleanolic acid:MβCD), and after stirring at 37°C for 48 hours, an MβCD-oleanolic acid (OA) complex having a final concentration of 10 mM was manufactured by filtering with a 0.45 µm syringe filter. After inserting a triterpenoid compound into the membrane by treating the manufactured MβCD-OA complex to the GUV, a polyol was input into a buffer solution surrounding the GUV to induce osmotic pressure conditions. A morphology change of the GUV was observed in real-time using an LSM880 confocal laser scanning microscope (Carl Zeiss, 20°C), and images were analyzed with Fiji. The results are shown in FIG. 9.

From the results of FIG. 9, immediate crumpling and deformation occurred in the GUV containing a triterpenoid compound under osmotic pressure conditions due to polyol exposure, which reflects that bending rigidity of a membrane by a triterpenoid compound decreased. Also, it was confirmed that a morphology is partially recovered as osmotic equilibrium is achieved through polyol influx over time.

### [Test Example 10] Molecular arrangement confirmation test of triterpenoid within membrane through GIWAXS analysis

To confirm a molecular arrangement and short-range intermolecular order of a triterpenoid compound within a lipid bilayer, a Lipoid P75-3-based planar multilamellar lipid film was manufactured and grazing-incidence wide-angle X-ray scattering (GIWAXS) analysis was performed. Specifically, Lipoid P75-3 was dissolved in a chloroform:methanol mixed solvent (2:1, v/v) alone or together with hydrophobic ingredients such as cholesterol and madecassic acid. The manufactured solution was drop-cast in an amount of 40-80 µL each onto a pre-cleaned silicon wafer substrate (WSI0PR0029, iTASCO), and after drying overnight at room temperature in a laminar flow hood, complete solvent removal was confirmed by further drying under vacuum for 12 hours or more.

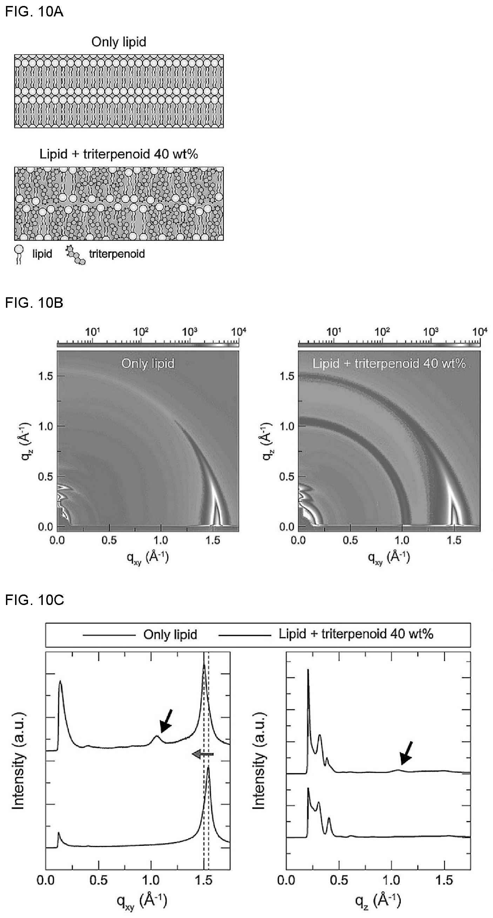

GIWAXS measurement was performed at the PLS-II 9A U-SAXS beamline (Pohang Accelerator Laboratory, Korea), and irradiation was performed for 5-10 seconds under conditions of an incident angle of 0.12° and a photon energy of 11.06 keV. An SX165 2D CCD was used as a detector, and a detector-specimen distance was set to 220 mm. A scattering angle was calibrated using sucrose crystals, and data analysis was performed with Igor-Pro software. The results are shown in FIGS. 10B and 10C.

FIG. 10A shows schematic diagrams of molecular arrangements within a multilamellar lipid film in a case not including a triterpenoid (top; Only lipid) and a case including the same (bottom; Lipid + triterpenoid 40 wt%). As shown in FIG. 10A, when a triterpenoid is inserted, lipid chain alignment is disturbed, and local curvature is formed in a membrane. From the results of FIGS. 10B and 10C, it was confirmed that an alkyl chain diffraction peak widens and isotropy increases as a triterpenoid compound is inserted. Also, it was confirmed that lipid alkyl chain packing became loose as a main lipid peak moved from q ≈ 1.53 Å⁻¹ to 1.50 Å⁻¹, and from a fact that a new isotropic peak (q ≈ 1.05 Å⁻¹, spacing ≈ 0.6 nm) appeared, it was confirmed that short-range intermolecular order is formed between triterpenoid molecules as the compound is inserted within the membrane.

### [Test Example 11] Behavior confirmation test of triterpenoid within membrane through molecular dynamics (MD) simulation

A molecular arrangement, distribution, tilt angle, and hydrogen bonding behavior of a triterpenoid compound within a lipid bilayer were confirmed through all-atom molecular dynamics (MD) simulation.

Specifically, a lipid bilayer system (including an MA-free control group for comparison) composed of DPPC (1,2-dipalmitoyl-sn-glycero-3-phosphocholine), cholesterol, and madecassic acid (MA) was constructed with CHARMM-GUI (rectangular box 5.5 nm × 5.5 nm × 8.0 nm, about 30 water molecules per lipid molecule, CHARMM36 force field, MA parameters generated by CHARMM-GUI Ligand Reader & Modeler, TIP3P water model). An initial packing density was constructed with area fractions of 0.7, 0.8, and 0.9, then 0.8 was selected (area per lipid 0.42 nm², consistent with experimental values). Simulation was performed with GROMACS v2021.4, and was respectively performed at 305 K and 353 K under conditions of energy minimization (steepest descent method) → 1.0 ns NVT (V-rescale thermostat, 1.0 ps time constant) → 5.0 ns NPT equilibration (C-rescale semi-isotropic barostat, 1 atm, 2.0 ps time constant) → 500 ns production run (Nose-Hoover thermostat 2.0 ps, Parrinello-Rahman semi-isotropic barostat 5.0 ps, periodic boundary conditions, 2 fs integration time interval, hydrogen atom bond LINCS constraints, electrostatic/van der Waals interaction calculation by PME method, cutoff distance 1.2 nm). Simulation data analysis included calculation of tilt angle distribution, radial distribution function (RDF), area per lipid (APL), density profile, and a number of hydrogen bonds. The results are shown in FIGS. 11A to 11D.

From the results of FIGS. 11A to 11D, it was confirmed that a triterpenoid (madecassic acid) is preferentially located at a lipid-water interface while having a specific orientation, and has a tendency to be distributed more asymmetrically in an outer leaflet of a bilayer. Also, from a fact that a peak appears near about 40° with respect to a membrane normal, it was confirmed that a conical geometry is formed, and an 0.8 nm dominant peak between triterpenoids was confirmed in RDF analysis, which becomes about 0.6 nm when projected onto a membrane plane, and this value is consistent with the GIWAXS signal, so that a presence of short-range in-plane ordering within the membrane plane was confirmed.

### [Test Example 12] Lateral diffusion restriction confirmation test of triterpenoid-containing membrane through FRAP analysis

To confirm whether hydrogen bonding network formation by a triterpenoid compound restricts lateral molecular diffusion within a membrane, a DOPC-based model lipid membrane was manufactured and a fluorescence recovery after photobleaching (FRAP) experiment was performed.

Specifically, a fluorescent lipid membrane was manufactured by including 0.5 mol% TR-DHPE (Texas Red 1,2-dihexadecanoyl-sn-glycero-3-phosphoethanolamine) in a DOPC-based lipid mixture, dissolving the same in chloroform, applying the same to a low-autofluorescence glass surface of a 35 mm confocal culture dish (SPL Life Sciences), and then drying overnight in a dust-free environment at room temperature. A triterpenoid-containing membrane was manufactured in the same manner by adding madecassic acid to a DOPC-based lipid mixture, and a triterpenoid-free membrane was used as a control group.

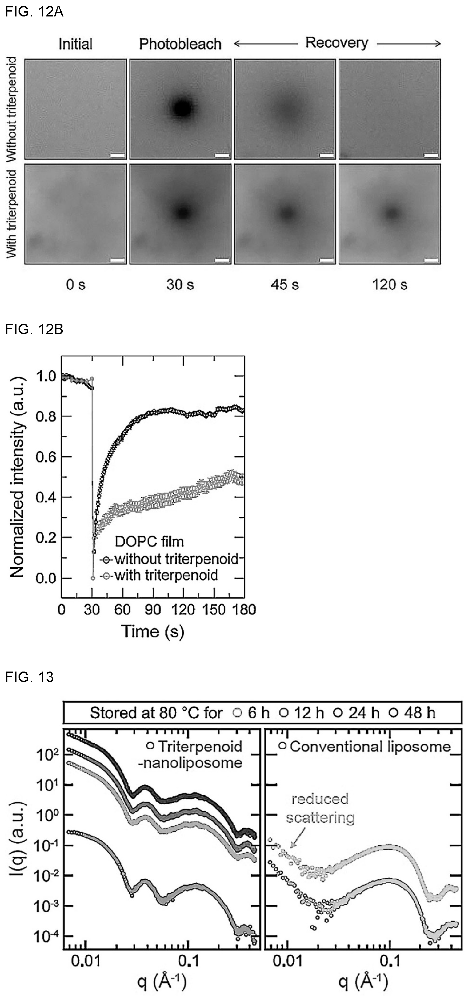

The FRAP experiment was performed at room temperature with an LSM880 confocal laser scanning microscope (Carl Zeiss, Airyscan detector, 100× oil immersion objective lens NA 1.57, KAIST KARA), and a 561 nm laser line (0.5% power during imaging, 100% power during photobleaching, ZEN Black software control) was used. A circular region of interest (1-2 µm) was photobleached after 30 frames of pre-bleaching until fluorescence almost disappeared, and afterward, recovery was monitored for up to 3 minutes at 1 frame per second with a 512×512 pixel frame (1 Airy unit pinhole). Intensity data was analyzed with the FRAP Profiler plugin of ImageJ (FIJI), and a recovery curve was generated by normalizing to a value before bleaching (1) and a minimum value immediately after bleaching (0). The results are shown in FIGS. 12A and 12B.

From the results of FIGS. 12A and 12B, it was confirmed that, while the DOPC lipid membrane not including a triterpenoid compound (without triterpenoid) almost completely recovers fluorescence intensity, the triterpenoid-containing membrane (with triterpenoid) recovers only about 50% of an original intensity. Such partial recovery indicates that the hydrogen bonding network formed by a triterpenoid compound significantly restricts lateral diffusion within a membrane. From this, it was confirmed that in the nanoliposome of the present disclosure, a triterpenoid compound improves membrane stability by forming a hydrogen bonding network within a lipid bilayer.

### [Test Example 13] Efficacy substance loading thermal stability confirmation test through calcein leakage test

According to the method of the above Manufacturing Example, calcein (using a buffer solution (10 mM HEPES, 50 mM NaCl, pH 7.4) containing 50 mM calcein as an aqueous phase) as a water-soluble fluorescent probe was encapsulated in the nanoliposome (Triterpenoid-nanoliposome) of Example 4 and a triterpenoid-free control group (Comparative Example 3; Conventional liposome) to confirm loading stability under thermal stress. Specifically, after manufacture of nanoliposomes, unencapsulated calcein was removed with a PD-10 column (Cytiva) (200 µL specimen loading, elution with 3.5 mL buffer solution, void volume collection). Fluorescence intensity over time of the purified nanoliposome was measured in an 80°C spectrofluorometer (Fluorolog-QM, HORIBA) under conditions of excitation 490 nm and emission 520 nm. Maximum leakage fluorescence (Fmax) was measured by Triton X-100 (0.1%) treatment. Calcein leakage (%) was calculated by the following formula: Leakage(%) = [(Ft - F0)/(Fmax - F0)] × 100. The results are shown in FIG. 13.

From the results of FIG. 13, it was confirmed that, under 80°C thermal stress conditions, while about 80% of encapsulated calcein leaked in Comparative Example 3 (general liposome), the triterpenoid nanoliposome of Example 4 maintained 95% or more of calcein in an encapsulated state. This shows that the hydrogen bonding network formed by a triterpenoid compound effectively maintains a membrane structure even under high-temperature stress conditions, thereby remarkably inhibiting leakage of an encapsulated substance.

### [Test Example 14] Long-term stability and freeze-thaw stability confirmation test

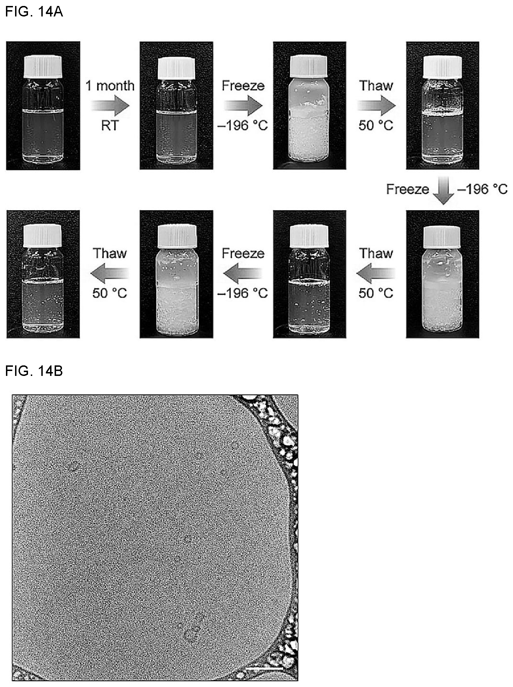

Regarding the nanoliposome of Example 4 manufactured in the above Manufacturing Example, stability under room temperature long-term storage and freeze-thaw conditions was confirmed. Specifically, for confirmation of long-term stability, a turbidity change was visually confirmed for a specimen stored at room temperature for 1 month. For confirmation of freeze-thaw stability, a process of freezing a specimen stored at room temperature for 1 month in liquid nitrogen (-196°C) for 5 minutes and thawing the same in a 50°C water bath for 5 minutes was repeated 3 times, and then a turbidity change and cryo-TEM images were confirmed. The results are shown in FIG. 14A (visual observation image) and FIG. 14B (cryo-TEM image after freeze-thaw treatment).

From the results of FIGS. 14A and 14B, it was confirmed that, in the nanoliposome of Example 4, a turbidity change was insignificant and a particle structure was maintained even after room temperature long-term storage, and particle distribution and structural integrity were maintained even after 3 freeze-thaw cycles. This shows that the nanoliposome of the present disclosure exhibits high structural stability even under long-term storage and temperature change conditions.

### [Test Example 15] Encapsulation efficiency and loading rate confirmation test of efficacy substance

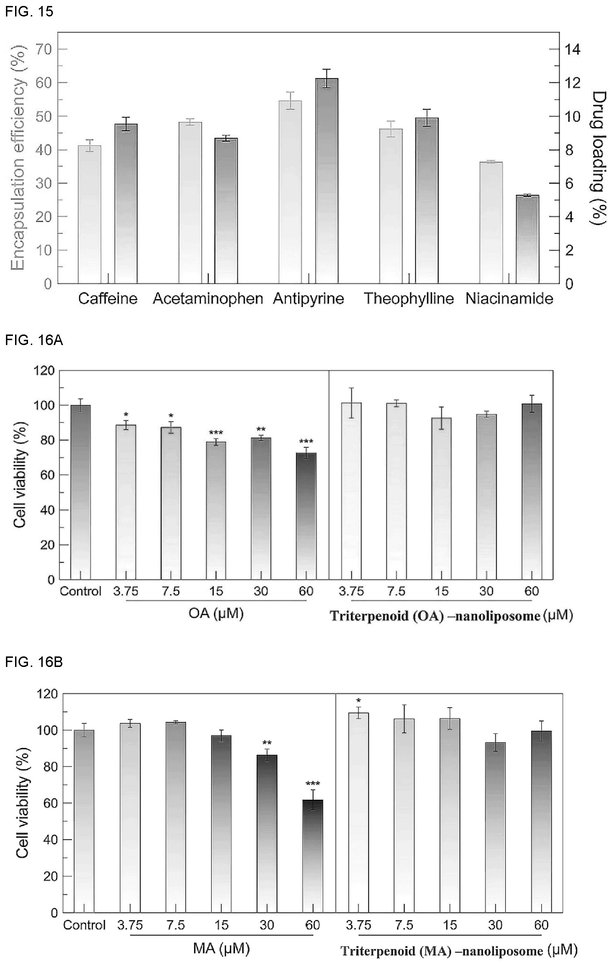

Encapsulation efficiency (EE, %) and efficacy substance loading rate (Drug Loading, DL, %) were confirmed by encapsulating efficacy substances (caffeine, acetaminophen, antipyrine, theophylline, niacinamide) in nanoliposomes. Specifically, in a state in which an efficacy substance is included in an aqueous phase, during manufacture of the nanoliposome of Example 4 according to the above Manufacturing Example, the aqueous phase was heated to 80°C, and mixed with a lipid-polyol phase of 80°C to induce nanoliposome formation. After incubation at 80°C for 10 minutes, it was cooled to 25°C. Unencapsulated efficacy substance was removed with a PD-10 column (Cytiva). An amount of drug contained in a purified nanoliposome fraction was quantified with a UV/vis nano spectrophotometer (Nabi, MICRODIGITAL), and nanoliposomes were destroyed with Triton X-100 (1%) before measurement. Absorbance was measured at a characteristic wavelength of each efficacy substance (caffeine 273 nm, acetaminophen 245 nm, antipyrine 242 nm, theophylline 272 nm, niacinamide 262 nm). A nanoliposome without an efficacy substance was used as a blank, and a drug concentration was calculated with a calibration curve. It was calculated as: Encapsulation Efficiency (EE, %) = (Amount of encapsulated efficacy substance / Amount of initially input efficacy substance) × 100, Drug Loading (DL, %) = (Amount of encapsulated efficacy substance / Mass of lyophilized nanoliposome) × 100. The results are shown in FIG. 15.

From the results of FIG. 15, it was confirmed that the nanoliposome of Example 4 exhibits meaningful encapsulation efficiency and efficacy substance loading rate for various efficacy substances, which shows that the nanoliposome of the present disclosure can be effectively utilized even for loading of an efficacy substance.

### [Test Example 16] Cytotoxicity confirmation test

Cytotoxicity for nanoliposomes of Example 1 (containing oleanolic acid (OA); triterpenoid(OA)-nanoliposome) and Example 5 (containing madecassic acid (MA); triterpenoid(MA)-nanoliposome) manufactured in the above Manufacturing Example, and pure triterpenoid compounds (oleanolic acid (OA) and madecassic acid (MA)) was confirmed. Specifically, neonatal normal human dermal fibroblasts (NHDFn, Thermo Fisher Scientific) were cultured in DMEM (Lonza) (containing 10% fetal bovine serum, 100 U/mL kanamycin, 100 µg/mL streptomycin) under conditions of 37°C and a humidified 5% CO₂ incubator. Cells were dispensed into a 48-well plate at 3.0 × 10⁴ cells/well, and each test specimen was treated at an indicated concentration for 18 hours. Cell viability was measured with Cell Counting Kit-8 (CCK-8, Dojindo), and absorbance at 450 nm was recorded with a BioTek Synergy 2 plate reader (Agilent Technologies). Values were expressed as mean ± standard deviation (mean ± SD) with n=3, and statistical significance was analyzed by Student's t-test (*p<0.05, **p<0.01, ***p<0.001), and the results are shown in FIGS. 16A and 16B.

From the results of FIGS. 16A and 16B, it was confirmed that, while nanoliposome formulations of Examples 1 and 5 did not exhibit detectable cytotoxicity throughout a tested concentration range, pure triterpenoid compounds exhibited significant cytotoxicity in a concentration-dependent manner. This shows that a triterpenoid compound in a form encapsulated in a nanoliposome is advantageous in terms of cell safety.

### [Test Example 17] Membrane thickness change confirmation test according to oil addition

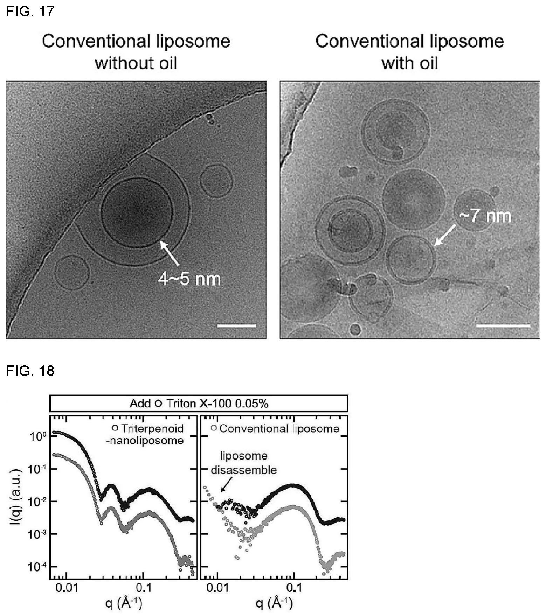

Using the oil (caprylic/capric triglyceride)-containing general liposome (Comparative Example 2; Conventional liposome with oil) and the oil-free general liposome (Comparative Example 3; Conventional liposome without oil) manufactured in the above Manufacturing Example, an effect of oil ingredient addition on a lipid bilayer thickness of a nanoliposome was confirmed through cryo-TEM analysis. The cryo-TEM analysis method is the same as in Test Example 4, and results thereof are shown in FIG. 17.

From the results of FIG. 17, it was confirmed that, in a case of the oil-containing liposome, a membrane thickness increases from about 4-5 nm to about 7 nm compared to the oil-free liposome, and from this, it was confirmed that an oil ingredient is inserted within a hydrophobic core of a lipid bilayer to increase a membrane thickness.

### [Test Example 18] SAXS-based nanoliposome structural stability confirmation test according to surfactant treatment

Regarding the nanoliposome (triterpenoid-nanoliposome) of Example 4 and the general liposome (conventional liposome) of Comparative Example 3, structural stability according to a treatment concentration of Triton X-100, which is a membrane-disrupting surfactant, was quantitatively confirmed through a SAXS scattering profile change.

Specifically, Triton X-100 was added to manufactured specimens in 0.05% (v/v) and stored at room temperature. SAXS measurement was performed under the same conditions as in Test Example 4, and disappearance of a characteristic peak in a scattering profile and a low-q scattering intensity change were analyzed. The results are shown in FIG. 18.

From the results of FIG. 18, in the general liposome of Comparative Example 3, a characteristic peak disappeared and a liposome structure collapsed even at a low Triton X-100 concentration of 0.05% (v/v), whereas in the nanoliposome of Example 4, only high-q shifts were observed while a peak position and form of a scattering profile were maintained under the same concentration condition, and from this, it was confirmed that in the nanoliposome of Example 4, only local rearrangement of lipids exists against a membrane-disrupting action of a surfactant, and structural integrity is preserved.

### [Manufacturing Example 2] Compound K-encapsulated nanoliposome

A Compound K-encapsulated nanoliposome (Example 6) was manufactured by the same method as in Manufacturing Example 1 with ingredients and contents according to Table 3 below.

**TABLE 3**

| Ingredient | | Content (wt%) |
|---|---|---|
| Polyol Part | Hydrogenated Lecithin | 4 |
| | Cholesterol | 2 |
| | Propylene Glycol | 2 |
| | Madecassic Acid | 2 |
| | Compound K | 60 |
| Distilled Water | | 30 |

### [Test Example 19] Stability confirmation test of Compound K-encapsulated nanoliposome

Regarding the Compound K-encapsulated nanoliposome manufactured in the above Manufacturing Example 2, stability was visually observed by storing the same at refrigeration (-10°C), room temperature (25°C), and high temperature (45°C, 60°C) for 1 week.

As a result, as shown in FIG. 21, it was confirmed that the nanoliposome encapsulating Compound K is stably maintained under various temperature conditions of refrigeration (-10°C), room temperature (25°C), and high temperature (45°C, 60°C).

### [Manufacturing Example 3] Cream composition including Compound K-encapsulated nanoliposome

A cream formulation composition including the Compound K-encapsulated nanoliposome manufactured in the above Manufacturing Example 2 was manufactured with ingredients and contents according to Table 4.

**TABLE 4**

| Ingredient | Content (wt%) |
|---|---|
| Cetearyl Alcohol | 1.0 |
| Glyceryl Stearate | 0.5 |
| Arachidyl Glucoside | 0.5 |
| Sorbitan Olivate | 1.0 |
| Squalane | 7.0 |
| Water | To 100 |
| Sodium Metaphosphate | 0.05 |
| Butylene Glycol | 10.0 |
| Hexanediol | 1.0 |
| Ethylhexylglycerin | 0.05 |
| Hydroxyethyl Acrylate/Sodium Acryloyldimethyl Taurate Copolymer | 1.1 |
| Compound K-encapsulated nanoliposome | 5 |

### [Test Example 20] Stability confirmation test of cream composition including Compound K-encapsulated nanoliposome

Regarding the cream formulation composition including the Compound K-encapsulated nanoliposome manufactured in the above Manufacturing Example 3, a hardness change was measured at 30°C at 1-week intervals for 4 weeks using a SUN RHEOMETER CR-100 instrument.

**TABLE 5**

| | Week 1 | Week 2 | Week 3 | Week 4 |
|---|---|---|---|---|
| Hardness of cream formulation (gf/cm²) | 33 | 34 | 34 | 36 |

As shown in Table 5 above, it was confirmed that the cream formulation composition including the Compound K-encapsulated nanoliposome is stably maintained without change over time at a level similar to a hardness (30 to 40 gf/cm²) of a general cream formulation not including nanoliposomes.

## Claims

1. A nanoliposome, comprising, as membrane components of the nanoliposome, a phospholipid; cholesterol; a polyol; and a triterpenoid compound.

2. The nanoliposome of claim 1, wherein the nanoliposome further comprises an oil as the membrane component.

3. The nanoliposome of claim 1 or 2, wherein a particle size of the nanoliposome is less than 30 nm, preferably, wherein a particle size of the nanoliposome is 15 nm or more and less than 30 nm.

4. The nanoliposome of any one of claims 1-3, wherein the triterpenoid compound is one or more selected from among asiaticoside, madecassic acid, asiatic acid, oleanolic acid, and ursonic acid, preferabley, wherein the triterpenoid compound is a combination of asiaticoside, madecassic acid, and asiatic acid.

5. The nanoliposome of any one of claims 1-4, wherein the phospholipid is contained in an amount of 0.001 to 5 wt% relative to a total weight of the nanoliposome.

6. The nanoliposome of any one of claims 1-5, wherein the cholesterol is contained in an amount of 0.0001 to 5 wt% relative to a total weight of the nanoliposome.

7. The nanoliposome of any one of claims 1-6, wherein the polyol is contained in an amount of 1 to 70 wt% relative to a total weight of the nanoliposome.

8. The nanoliposome of any one of claims 2-7, wherein the oil is contained in an amount of 0.001 to 10 wt% relative to a total weight of the nanoliposome.

9. The nanoliposome of any one of claims 1-8, wherein the triterpenoid compound is contained in an amount of 0.001 to 5 wt% relative to a total weight of the nanoliposome.

10. The nanoliposome of any one of claims 1-9, wherein the nanoliposome loads one or more efficacy ingredients selected from among caffeine, acetaminophen, antipyrine, theophylline, niacinamide, riboflavin, PDRN, vitamin C, Melasolv, Compound K, phytosterol, ceramide, and retinol.

11. A cosmetic composition comprising the nanoliposome of any one of claims 1-10.

12. A manufacturing method of the nanoliposome of any one of claims 1-10, comprising: a first mixing step of mixing a phospholipid, cholesterol, a polyol, and a triterpenoid compound; and a second mixing step of mixing a mixture obtained in the first mixing step with water.

13. The manufacturing method of claim 12, wherein, in the first mixing step, an oil is additionally mixed.

14. The manufacturing method of claim 12 or 13, wherein a mixing temperature in the first mixing step is 25 to 110°C,
optionally, wherein a temperature of the water is 25 to 110°C.

15. The manufacturing method of any one of claims 12-14, wherein, in the first mixing step, the polyol dissolves the phospholipid, the cholesterol, and the triterpenoid compound to form a multilamellar giant vesicle,
optionally, wherein, in the second mixing step, the multilamellar giant vesicle formed in the first mixing step is fragmented into a unilamellar nanoliposome by an osmotic pressure gradient due to mixing with water.
